# EUROPÄISCHE PATENTSCHRIFT

(11) **EP 1 663 943 B1**
(45) Veröffentlichungstag und Bekanntmachung des Hinweises auf die Patenterteilung: **27.12.2006**
(21) Anmeldenummer: 04764834.0
(22) Anmeldetag: 04.09.2004
(51) Int. Cl.: C07C 209/48, C07C 211/27

(54) **VERFAHREN ZUR HERSTELLUNG VON META-XYLYLENDIAMIN (MXDA)**
METHOD FOR PRODUCING META-XYLYLENEDIAMINE (MXDA)
PROCEDE DE PRODUCTION DE META-XYLYLENE DIAMINE (MXDA)

(30) Priorität: 10.09.2003 DE 10341612
(43) Veröffentlichungstag der Anmeldung: 07.06.2006
(73) Patentinhaber: BASF Aktiengesellschaft, 67056 Ludwigshafen (DE)
(72) Erfinder: HUGO, Randolf, 67246 Dirmstein (DE); JOURDAN, Sabine, 68163 Mannheim (DE); WENZ, Kirsten, 68161 Mannheim (DE); PREISS, Thomas, 67256 Weisenheim (DE); WECK, Alexander, 67251 Freinsheim (DE)
(86) Internationale Anmeldenummer: PCT/EP2004/009883
(87) Internationale Veröffentlichungsnummer: WO 2005/026102

(56) Entgegenhaltungen:
- EP-A- 1 193 244
- EP-A- 1 279 661
- DE-A- 2 041 360

## Beschreibung

Die vorliegende Erfindung betrifft ein Verfahren zur Herstellung von meta-Xylylendiamin umfassend die Schritte
Ammonoxidation von meta-Xylol zu Isophthalodinitril, wobei das dampfförmige' Produkt dieser Ammonoxidationsstufe direkt mit einem flüssigen organischen Lösungsmittel in Kontakt gebracht wird (Quench),
Abtrennung von Produkten mit einem Siedepunkt höher als Isophthalodinitril (Hochsiedern) aus der erhaltenen Quenchlösung oder -suspension und Hydrierung des Isophthalodinitrils.

Xylylendiamin (Bis(aminomethyl)benzol) ist ein nützlicher Ausgangsstoff, z.B. für die Synthese von Polyamiden,-Epoxyhärtern oder als Zwischenstufe zur Herstellung von Isocyanaten.

Die zweistufige Synthese von meta-Xylylendiamin (MXDA) durch Ammonoxidation von meta-Xylol und anschließender Hydrierung des erhaltenen Isophthalodinitrils (JPDN) ist bekannt.

EP-A2-1 113 001 (Mitsubishi Gas Chem. Comp.) beschreibt ein Verfahren zur Herstellung von Nitrilverbindungen durch Ammonoxidation entsprechender carbocyclischer oder heterocyclischer Verbindungen, wobei überschüssiger Ammoniak aus dem Reaktionsprodukt recycliert wird. Beschrieben wird auch das direkte in Kontakt bringen des dampfförmigen Produkts der Ammonoxidationsstufe mit einem flüssigen organischen Lösungsmittel, bei dem es sich insbesondere um aliphatische oder aromatische Kohlenwasserstoffe handelt. (Absätze [0045] und [0046]).

EP-A2-1 193 247 und EP-A1-1 279 661 (beide Mitsubishi Gas Chem. Comp.) betreffen ein Verfahren zur Reinigung von iso-Phthalodinitril (IPDN) bzw. ein Verfahren zur Herstellung von reinem XDA, in dem das Phthalodinitril durch Ammonoxidation von Xylol synthetisiert wird, wobei das dampfförmige Produkt der Ammonoxidationsstufe direkt mit einem flüssigen organischen Lösungsmittel in Kontakt gebracht wird (Quench). Das organische Lösungsmittel ist ausgewählt aus Alkylbenzole, heterocyclische Verbindungen, aromatische Nitrile und heterocyclische Nitrile und hat einen Siedepunkt, der unter dem von Phthalodinitril liegt (EP-A2-1 193 247: Spalte 4, Absatz [0018] und [0019]; EP-A1-1 279 661: Spalten 4-5, Absatz [0023] und [0024]).

EP-A2-1 193 244 (Mitsubishi Gas Chem. Comp.) beschreibt ein Verfahren zur Herstellung von XDA durch Hydrierung von Phthalodinitril, welches in einer vorherigen Stufe durch Ammonoxidation von Xylol synthetisiert wird, wobei das dampfförmige Produkt der Ammonoxidationsstufe direkt mit einem flüssigen organischen Lösungsmittel in Kontakt gebracht wird (Quench) und die erhaltene Quenchlösung oder -suspension der Hydrierung zugeführt wird.
Bevorzugte organische Lösungsmittel sind C₆-C₁₂ aromatische Kohlenwasserstoffe, wie Xylol und Pseudocumol (Spalte 6, Absatz [0027] und [0028]).

DE-A-21 64 169 beschreibt auf Seite 6, letzter Absatz, die Hydrierung von IPDN zu meta-XDA in Gegenwart eines Ni- und/oder Co-Katalysators in Ammoniak als Lösungsmittel.

Fünf parallele BASF-Patentanmeldungen mit jeweils gleichem Anmeldetag betreffen jeweils Verfahren zur Herstellung von XDA.

Der vorliegenden Erfindung lag die Aufgabe zugrunde, ein verbessertes wirtschaftliches Verfahren zur Herstellung von hoch reinem meta-Xylylendiamin, mit hoher Ausbeute und Raum-Zeit-Ausbeute (RZA) aufzufinden, welches bei mit bekannten Verfahren, z.B. dem Verfahren gemäß EP-A2-1 193 244, vergleichbaren Durchsätzen aufgrund verringerter Stoffströme, insbesondere Lösungsmittelströme, inkl. Rückführströme, verkleinerte Apparate und Maschinen ermöglicht.

Demgemäß wurde ein Verfahren zur Herstellung von meta-Xylylendiamin umfassend die Schritte
Ammonoxidation von meta-Xylol zu Isophthalodinitril, wobei das dampfförmige Produkt dieser Ammonoxidationsstufe direkt mit einem flüssigen organischen Lösungsmittel in Kontakt gebracht wird (Quench),
Abtrennung von Produkten mit einem Siedepunkt höher als Isophthalodinitril (Hochsiedern) aus der erhaltenen Quenchlösung oder -suspension und
Hydrierung des Isophthalodinitrils gefunden,
welches dadurch gekennzeichnet ist, dass es sich bei dem für den Quench verwendeten organischen Lösungsmittel um N-Methyl-2-pyrrolidon (NMP) handelt,
nach der Abtrennung der Hochsieder und vor der Hydrierung eine teilweise oder vollständige Abtrennung des NMPs und/oder von Produkten mit einem Siedepunkt niedriger als Isophthalodinitril (Leichtsieder) erfolgt und
das Isophthalodinitril für den Hydrierschritt in einem organischen Lösungsmittel oder in flüssigem Ammoniak gelöst oder suspendiert ist.

Das erfindungsgemäße Verfahren lässt sich wie folgt ausführen:

### Ammonoxidationsstufe:

Die Ammonoxidation von meta-Xylol zum entsprechenden Isophthalodinitril wird im allgemeinen nach dem Fachmann bekannten Verfahren durchgeführt.

Die Ammonoxidation des Methylaromaten wird bevorzugt durchgeführt an einem Multioxidkatalysator mit Ammoniak und einem sauerstoffhaltigen Gas (Sauerstoff oder Luft oder beides) in einem Wirbelschichtreaktor oder einem Rohr(bündel)reaktor.

Die Reaktionstemperatur liegt dabei im allgemeinen bei 300 bis 500°C, bevorzugt bei 330 bis 480°C.

Der Katalysator enthält bevorzugt V, Sb und/oder Cr und setzt sich besonders bevorzugt zusammen aus [V, Sb und Alkalimetallen] oder [V, Cr, Mo und B] jeweils als Vollkatalysator oder auf einem inerten Träger.
Als inerter Träger sind bevorzugt SiO₂, Al₂O₃ oder ein Gemisch der beiden oder Steatit.

Solch eine Verfahrensweise ist z.B. in den BASF-Patentanmeldungen EP-A-767165 und EP-A-699 476 beschrieben, auf die hiermit ausdrücklich Bezug genommen wird.

Auch die BASF-Patentanmeldungen EP-A-222 249, DE-A-35 40 517 und DE-A-37 00 710 offenbaren geeignete Ammonoxidationskatalysatoren.

Die Ammonoxidation kann auch gemäß den in den eingangs zitierten Anmeldungen EP-A2-1 113 001, EP-A2-1 193 247, EP-A1-1 279 661 und EP-A2-1 193 244 beschriebenen Verfahren durchgeführt werden.

### Quench:

Der bei der Ammonoxidation produzierte Dampf, enthaltend das Wertprodukt Isophthalodinitril, wird direkt mit dem flüssigen organischen Lösungsmittel N-Methyl-2-pyrrolidon (NMP) in Kontakt gebracht (Quench mit NMP als Quenchflüssigkeit, Quenchmittel).

Das für den Quench verwendete NMP kann auch bereits gelöstes oder suspendiertes Isophthalodinitril enthalten.

Durch die plötzliche Temperaturabsenkung beim in Kontakt bringen des dampfförmigen Isophthalodinitrils mit dem flüssigen Lösungsmittel NMP (Quench) wird die Bildung von unerwünschten Neben- und Zersetzungsprodukten, die zur Qualitätsminderung des Isophthalodinitrils und schließlich des MXDAs führen, verringert.

Das dampfförmige Isophthalodinitril wird durch den Quench direkt in das flüssige Lösungsmittel NMP aufgenommen, wobei eine Lösung und/oder eine Suspension entsteht, welche unmittelbar weiter verarbeitet werden kann.

Als Frischzulauf wird im allgemeinen technisches NMP mit einer Reinheit > 99 Gew.-%, insbesondere > 99,5 Gew.-%, eingesetzt.
Bevorzugt kann aus dem Verfahren zurückgewonnenes NMP als Quenchflüssigkeit eingesetzt werden. Hier kann die Reinheit der Quenchflüssigkeit auch ≤ 99 Gew.-%, z.B. 90-98 Gew.-%, betragen, insbesondere dann, wenn es sich nicht um verfahrensfremde Substanzen (also u.a. um Wasser, Ammoniak, Benzonitril, Tolunitril, Xylol, o-, m- oder p-Methyl-benzylamin, Benzylamin, Xylylendiamin) als Verunreinigungen handelt.

Die Menge des verwendeten Lösungsmittels NMP ist im allgemeinen so bemessen, dass Lösungen/Suspensionen mit einem Phthalodinitril-Gehalt von 15 bis 75 Gew.-%, bevorzugt 25 bis 60 Gew.-%, erhalten werden.

Die Einleitung des dampfförmigen Austrags der Ammonoxidation, enthaltend das Isophthalodinitril (IPDN), in das flüssige NMP erfolgt in einem Quenchapparat, z.B. bevorzugt in einem Fallfilmkondensator (Dünnschicht-, Rieselfilm- oder Fallstromkondensator), in einem Düsenapparat oder in einer Kolonne. Dabei kann das dampfförmige Isophthalodinitril im Gleich- oder im Gegenstrom mit dem flüssigen Lösungsmittel geführt werden. Bei Gleichstromführung wird das dampfförmige Isophthalodinitril von oben in den Quenchapparat eingeleitet. Vorteilhaft ist die tangentiale Zufuhr des flüssigen Lösungsmittels am Kopf des Fallfilmkondensators oder die Zufuhr des flüssigen Lösungsmittels durch eine oder mehrere Düsen um eine vollständige Benetzung der Innenwand des Quenchapparates zu erreichen.

Im Falle einer Quenchkolonne wird das Gas aus der Ammonoxidation am Kolonnensumpf aufgegeben und das Lösungsmittel am Kopf zugeführt. Der Quenchapparat kann zur Vergrößerung der zur Kondensation verfügbaren Oberfläche mit Einbauten wie Böden, geordneten Packungen oder ungeordneten Schüttungen ausgerüstet sein.

Das NMP für den Quench kann im einmaligen Durchlauf oder als Kreislaufflüssigkeit eingesetzt werden.

Vorteilhafterweise wird ein Teil der Quenchlösung oder -suspension im Kreis gefahren. Mittels eines im Kreislauf eingebauten Wärmeüberträgers wird die Quenchlösung oder -suspension gekühlt.
Dabei werden die Temperatur des Kreislaufmediums und der Kreislaufmengenstrom so eingestellt und aufeinander abgestimmt, dass die gewünschte Temperatur im Quenchaustritt erreicht wird. Die Temperatur des Kreislaufmediums wird umso niedriger gewählt, je kleiner der Mengenstrom des Kreislauf mediums ist und umgekehrt, wobei Löslichkeiten und Schmelzpunkte sowie die hydraulischen Belastungsgrenzen des Quenchapparates zu berücksichtigen sind.

Der Mengenstrom des frisch zulaufenden NMPs ist von der Quenchtemperatur abhängig. Er wird so eingestellt, dass die gewünschte Konzentration der PDN-Lösung oder - suspension erhalten wird. Da die Löslichkeit von IPDN in NMP mit zunehmender Temperatur ansteigt, kann mit zunehmender Quenchaustrittstemperatur eine höhere IPDN-Konzentration im NMP gefahren werden.

Das Kreislaufmedium wird gemeinsam mit dem frischen Lösungsmittel oder davon getrennt an geeigneter Stelle des Quenchapparats zugefahren.
Im Falle einer im Gegenstrom betriebenen Quenchkolonne wird das frische NMP am Kopf und das Kreislauf medium weiter unten, etwa in Kolonnenmitte zugefahren.

Im allgemeinen wird durch Temperierung des eingesetzten NMPs und/oder des Kreislaufmediums die Temperatur des flüssigen Quenchaustrags auf 40 bis 180°C, bevorzugt 50 bis 120°C, insbesondere 80 bis 120°C, eingestellt.

Der Siedepunkt von Phthalodinitril liegt im Bereich von 1 bis 100 mbar um ca. 60 Kelvin über dem Siedepunkt von NMP.

Der Absolutdruck beim Quenchen beträgt im allgemeinen 0,5 bis 1,5 bar. Bevorzugt wird bei leichtem Überdruck gefahren.

Xylol, Wasser, NH₃, CO₂, N₂ etc., die im dampfförmigen Austrag der Ammonoxidation in der Regel enthalten sind, werden unter Quenchbedingungen im Quench-Lösungsmittel NMP nur teilweise oder praktisch nicht gelöst und werden aus dem Quench-Apparat überwiegend gasförmig abgetrennt.

Abtrennung von Produkten mit einem Siedepunkt höher als Isophthalodinitril (bei gleichem Druck) (Hochsiedern) aus der erhaltenen Quenchlösung oder -suspension: Die Abtrennung von Hochsiedem aus der erhaltenen Quenchlösung oder -suspension erfolgt bevorzugt destillativ.

Die Abtrennung von Hochsiedern aus der erhaltenen Quenchlösung oder -suspension kann in einer oder mehreren hintereinander geschalteten Verdampferstufen oder in einer Destillationskolonne erfolgen, wobei die Hochsieder über Sumpf ausgeschleust werden, während Isophthalodinitril zusammen mit dem Lösungsmittel NMP und Leichtsiedern über Kopf abgetrennt werden.

Bevorzugt wird zur Hochsiederabtrennung eine Destillationskolonne verwendet.

Die Kolonne ist vorzugsweise mit den üblichen Einbauten zur Erhöhung der Trennleistung, wie Böden, geordnete oder ungeordnete Packungen, etc., ausgerüstet.

Die Auslegung der Kolonne (insbesondere Zahl der Trennstufen, Zulaufstelle, Rücklaufverhältnis, etc.) kann, abgestimmt auf die jeweilige Zusammensetzung der Lösung, durch den Fachmann nach ihm geläufigen Methoden vorgenommen werden. Bevorzugt wird unter vermindertem Druck gefahren, um die Sumpftemperatur zu begrenzen.

Teilweise oder vollständige Abtrennung des NMPs und/oder von Produkten mit einem Siedepunkt niedriger als Isophthalodinitril (bei gleichem Druck) (Leichtsieder):

Je niedriger die Temperatur im Quenchschritt ist, desto höher ist der Anteil von Wasser und tiefer als IPDN siedenden Nebenkomponenten (bei gleichem Druck) (z.B. Benzonitril, Tolunitril) im flüssigen Quenchaustrag.

Im erfindungsgemäßen Verfahren werden vor der Hydrierung des Phthalodinitrils aus der erhaltenen Quenchlösung oder -suspension nach der Hochsiederabtrennung Wasser und Produkte mit einem Siedepunkt niedriger als Isophthalodinitril (bei gleichem Druck) (Leichtsieder; z.B. nicht umgesetztes Xylol, Benzonitril, Tolunitril, jeweils als Heteroazeotrop mit Wasser, Wasser, Benzonitril, Tolunitril; Aufzählung mit zunehmenden Siedepunkt (bei gleichem Druck); wie ggf. auch Benzylamin, o-, m-, p-Methyl-benzylamin, Xylylendiamine, wobei diese Amine aus zurückgeführten Lösungsmittel von der Hydrierstufe stammen) teilweise oder vollständig abgetrennt. Diese Abtrennung erfolgt bevorzugt destillativ.

Auch das im Quench verwendete NMP kann in diesem Schritt als Leichtsieder teilweise oder vollständig abgetrennt werden.

Diese Abtrennung des NMPs und/oder der Leichtsieder kann in einer oder mehreren hintereinander geschalteten Verdampferstufen oder in einer Destillationskolonne über Kopf erfolgen.

Bevorzugt wird eine Destillationskolonne verwendet, welche vorzugsweise mit den üblichen Einbauten zur Erhöhung der Trennleistung, wie Böden, geordnete oder ungeordnete Packungen, etc., ausgerüstet ist.

Die Auslegung der Kolonne (insbesondere Zahl der Trennstufen, Zulaufstelle, Rücklaufverhäftnis, etc.) kann, abgestimmt auf die jeweilige Zusammensetzung der Lösung oder Suspension, durch den Fachmann nach ihm geläufigen Methoden vorgenommen werden.

Bevorzugt wird unter vermindertem Druck gefahren, um die Sumpftemperatur zu begrenzen.

Kombination der Leichtsieder- und Hochsieder-Abtrennung in einer Seitenabzugskolonne, insbesondere Trennwandkolonne, mit Seitenabzug:

Die Abtrennung von Hochsiedem aus der erhaltenen Quenchlösung oder -suspension über Sumpf und die Abtrennung des NMPs und/oder von Leichtsiedern über Kopf erfolgt besonders bevorzugt in einer einzigen Kolonne, die als Seitenabzugskolonne ausgestaltet ist.
Dabei wird das Isophthalodinitril flüssig aus einem Seitenabzug im Verstärkungsteil oder dampfförmig aus einem Seitenabzug im Abtriebsteil der Kolonne abgezogen.

Die Auslegung der Kolonne (insbesondere Zahl der Trennstufen, Zulaufstelle, Rücklaufverhältnis, Lage des Seitenabzugs, etc.) kann, abgestimmt auf die jeweilige Zusammensetzung der Lösung, durch den Fachmann nach ihm geläufigen Methoden vorgenommen werden.

Bevorzugt wird unter vermindertem Druck (z.B. 30 bis 250 mbar (abs.), insbesondere 50 bis 100 mbar (abs.)) gefahren, um die Sumpftemperatur zu begrenzen.

In einer weiteren besonderen Verfahrensausgestaltung erfolgt die Abtrennung von Hochsiedem aus der erhaltenen Quenchlösung oder -suspension über Sumpf und die Abtrennung des NMPs und/oder von Leichtsiedern über Kopf in einer einzigen Kolonne, die als Trennwandkolonne mit Seitenabzug ausgestaltet ist.
Das Isophthalodinitril wird dabei flüssig aus einem Seitenabzug im Bereich der Trennwand abgezogen.

Geeignete Trennwandkolonnen sind dem Fachmann z.B. aus Hydrocarbon Processing, März 2002, Seite 50 B - 50 D; EP-A-1 040 857, DE-A1-101 00 552,
WO-A-02/40434, US 4,230,533, EP-A1-638 778, EP-A1-1 181 964, WO-A-02/45811, EP-A1-1 205 460, DE-A1-198 13 720, EP-A1-1 084 741, bekannt.

### Hydrierung:

Zur Hydrierung des Isophthalodinitrils zum entsprechenden meta-Xylylendiamin wird das gemäß der obigen Schritte erhaltene IPDN optional in einem organischen Lösungsmittel oder in flüssigem Ammoniak gelöst oder suspendiert.

Bevorzugte Lösungsmittel sind NMP, Xylol, Benzylamin, o-, m- oder p-Methyl-benzylamin, Xylylendiamin und Mischungen hiervon. Wurde im Schritt der NMP-und/oder Leichtsiederabtrennung das NMP nicht oder nur teilweise abgetrennt, so kann die erhaltene Lösung oder Suspension von IPDN in NMP in die Hydrierung gefahren werden.

Für die Hydrierung des Isophthatodinitrils zum meta-Xylylendiamin wird der Lösung oder Suspension in einem organischen Lösungsmittel besonders bevorzugt Ammoniak, bevorzugt in flüssiger Form, zugefügt. Das Hinzufügen des Ammoniaks kann direkt nach der Stufe der NMP- und/oder Leichtsiederabtrennung oder erst in der Hydrierstufe erfolgen.

Das Gewichtsverhältnis von Dinitril zu Ammoniak beträgt hierbei im Frischzulauf im allgemeinen 1 : 0,15 bis 1 : 15, vorzugsweise 1 : 0,5 bis 1 : 10, besonders bevorzugt 1 : 1 bis 1 : 5.

Für die Hydrierung können die dem Fachmann für diese Umsetzung bekannten Katalysatoren und Reaktoren (z.B. Festbett- oder Suspensionsfahrweise) sowie Verfahren (kontinuierlich, halbkontinuierlich, diskontinuierlich) angewendet werden.

Bei der Katalysatorfestbettfahrweise ist sowohl die Sumpf- als auch die Rieselfahrweise möglich. Bevorzugt ist eine Rieselfahrweise.

Diesbezüglich wird hiermit z.B. auf die in den Anmeldungen GB-A-852,972 (Äquivalent: DE-A-11 19 285) (BASF AG) und DE-A-12 59 899 (BASF AG) und dem US Patent Nr. 3,069,469 (California Research Corp.) beschriebenen Verfahren verwiesen.

Der Hydrierreaktor kann in geradem Durchgang gefahren werden. Alternativ ist auch eine Kreislauffahrweise möglich, bei der ein Teil des Reaktoraustrages an den Reaktoreingang zurückgeführt wird, bevorzugt ohne vorherige Aufarbeitung des Kreislaufstromes. Damit lässt sich eine optimale Verdünnung der Reaktionslösung erreichen, was sich günstig auf die Selektivität auswirkt. Insbesondere kann der Kreislaufstrom mittels eines externen Wärmeüberträgers auf einfache und kostengünstige Weise gekühlt und somit die Reaktionswärme abgeführt werden. Der Reaktor lässt sich dadurch auch adiabat betreiben, wobei der Temperaturanstieg der Reaktionslösung durch den gekühlten Kreislaufstrom begrenzt werden kann. Da der Reaktor selbst dann nicht gekühlt werden muss, ist eine einfache und kostengünstige Bauform möglich. Eine Alternative stellt ein gekühlter Rohrbündelreaktor dar.

Bevorzugt sind Katalysatoren, die Kobalt und/oder Nickel und/oder Eisen, als Vollkatalysator oder auf einem inerten Träger, enthalten.

Hierbei liegen die Reaktionstemperaturen im allgemeinen bei 40 bis 150°C, bevorzugt bei 40 bis 120°C.

Der Druck liegt im allgemeinen bei 40 bis 300 bar, bevorzugt 100 bis 200 bar.

### Isolierung des MXDAs:

Nach der Hydrierung werden das gegebenenfalls verwendete Lösungsmittel und der gegebenenfalls eingesetzte Ammoniak abdestilliert.

Bevorzugt erfolgt eine Reinigung des meta-Xylylendiamins durch Abdestillation leichtersiedender Nebenprodukte (bei gleichem Druck) über Kopf und destillativer Abtrennung von schwerersiedenden Verunreinigungen über Sumpf.

Besonders bevorzugt ist die Fahrweise, in der man nach der Hydrierung das Lösungsmittel, gegebenenfalls Ammoniak sowie gegebenenfalls leichtersiedende Nebenprodukte über Kopf abdestilliert und danach schwerersiedende Verunreinigungen vom meta-Xylylendiamin destillativ über Sumpf abtrennt.

In einer besonderen Ausführungsform kann die Abtrennung leichter- und schwerersiedender Nebenprodukte auch in einer Seitenabzugs- oder Trennwandkolonne erfolgen, wobei reines meta-Xylylendiamin über einen flüssigen oder gasförmigen Seitenabzug gewonnen wird.

Je nach gewünschter Reinheit wird das Produkt (MXDA) zusätzlich mit einem organischen Lösungsmittel, bevorzugt einem aliphatischen Kohlenwasserstoff, insbesondere einem cycloaliphatischen Kohlenwasserstoff, ganz besonders Cyclohexan oder Methylcyclohexan, extrahiert.
Diese Reinigung durch Extraktion kann z.B. gemäß DE-A-1 074 592 erfolgen.

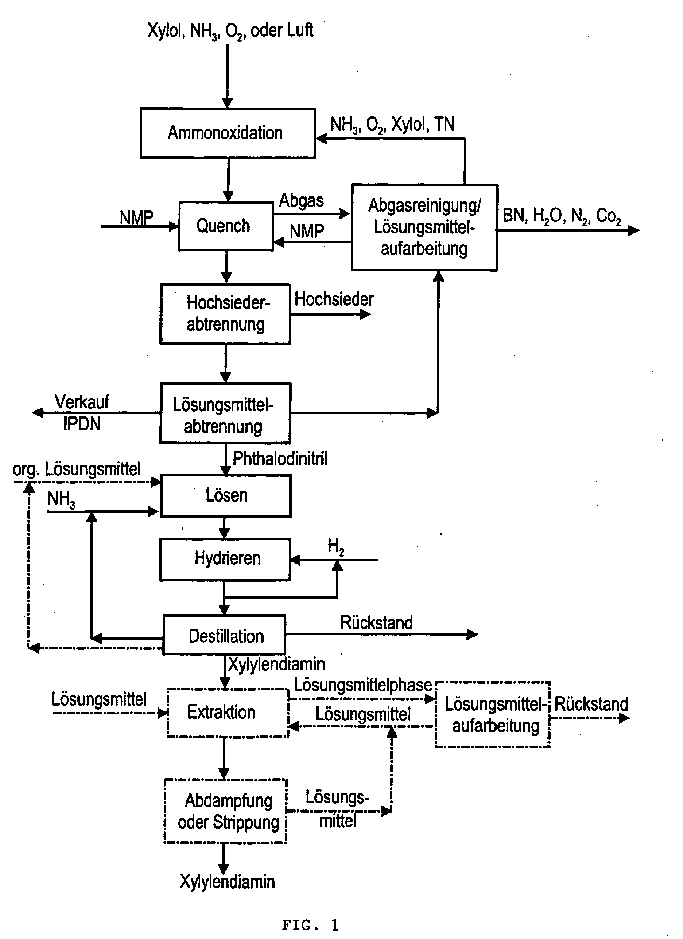

Einen schematischen Überblick über eine bevorzugte Ausgestaltung des erfindungsgemäßen Verfahrens gibt die Abbildung 1 in der Anlage.
Die optionalen Verfahrensschritte zum Lösungsmittelkreislauf in der Hydrierung und zur ,extraktiven XDA-Reinigung' sind gestrichelt gezeichnet.

### Beispiele

### Beispiel 1:

Ammonoxidation von m-Xylol, anschließendes Quenchen der Reaktionsgase mit NMP als Lösungsmittel und Hydrierung des in der Ammonoxidationsstufe entstandenen IPDNs (vergl. Verfahrensschema in Abbildung 1).

Ein Katalysator der Zusammensetzung V₄Sb₃W_{0,4}Cs_{0,2}auf Steatit wurde als Festbett in einen Rohrreaktor eingebaut. Die Apparatur wurde von außen auf 400°C aufgeheizt. Dem Reaktor wurde verdampftes m-Xylol, gasförmiger Ammoniak, Luft und Stickstoff zugefahren (NH₃ / m-Xylol = 8 mol / 1 mol; O₂ / m-Xylol = 4 mol / 1 mol). Der vordere Teil des Reaktors war mit einer Inertschüttung gefüllt, so dass die Einsatzstoffe vorgemischt und auf 400°C vorgeheizt die Reaktionszone erreichten. Im Reaktor herrschte ein leichter Überdruck von 0,02-0,03 bar. Die Hot-Spot-Temperatur erreichte 450°C. Man erhielt bei einem Umsatz (U) von m-Xylol von 79 % eine Selektivität (S) zu IPDN von 68 %.

Das aus dem Reaktor austretende Gasgemisch wird in einer Kolonne mit NMP gequencht. Aus der Quenchkolonne wird bei 120°C eine Lösung von IPDN in NMP ausgetragen, welche 0,6 Gew.-% m-Xylol, 1,7 Gew.-% Wasser, 0,1 Gew.-% Benzonitril, 3,4 Gew.-% Tolunitril, 19 Gew.-% IPDN und ca. 75 Gew.-% NMP enthält. Über Kopf der Quenchkolonne werden nicht umgesetzte Reaktionsgase und Inertgase sowie nicht umgesetztes m-Xylol sowie etwas NMP gasförmig abgezogen. Dieses Gas kann aufgearbeitet werden, um die Wertstoffe (insbesondere NH₃, m-Xylol, NMP sowie Tolunitril) in die Reaktionsstufe bzw. in den Quenchkreis zurückzuführen. Inerte und Begleitkomponenten (H₂O, Benzonitril, N₂, CO₂, etc.) werden aus der Aufarbeitungsstufe ausgeschleust.

Die nach dem Quench erhaltene Lösung von IPDN in NMP wird bei 70 mbar (abs.) zum Kopf einer Abtriebskolonne gefahren, in der über Sumpf hochsiedende Nebenkomponenten abgetrennt werden. Über Kopf werden NMP, IPDN sowie die noch enthaltenen leichtsiedenden Nebenkomponenten (Xylol, Tolunitril, Benzonitril, etc.) abgezogen. Die Sumpftemperatur der Kolonne beträgt 200°C, die Kopftemperatur 140°C. Der Kopfabzugsstrom wird direkt, d.h. ohne Kondensation auf eine der mittleren Stufen einer zweiten Kolonne gefahren, welche ebenfalls bei 70 mbar (abs.) betrieben wird. Über Sumpf wird IPDN in einer Reinheit von mehr als 99,9 % abgezogen, während Lösungsmittel und Nebenkomponenten über Kopf abgetrennt werden. Die Sumpftemperatur dieser Kolonne beträgt 185°C.

Eine Mischung bestehend aus 15 Gew.-% IPDN und 85 Gew.-% MXDA, die aus den reinen Komponenten zusammengemischt wurde, wurde in einem kontinuierlich betriebenen 70 ml-Rohrreaktor an einem Kobalt-Vollkontakt bei 60°C und 190 bar hydriert. Über den Katalysator wurden stündlich 117 g IPDN-Lösung sowie 150 g Ammoniak geleitet. Ein Viertel der Volumenmenge wurde als Lösungsmittel im Kreis gefahren. Die Ausbeute an MXDA betrug 92 % bezogen auf eingesetztes IPDN.

In anschließenden Destillationsschritten wurden zuerst Ammoniak bei 190°C und die leicht- und hochsiedenden Verunreinigungen in einer Batchdestillation bei einem Kopfdruck von 57 mbar und einer Sumpftemperatur von ca. 180°C abgetrennt. MXDA wurde in einer Reinheit von mehr als 99,9 Gew.-% erhalten.

(Die oben angegebenen Daten des Quenchschrittes und der Destillationen sind die Ergebnisse einer thermodynamischen Simulation. Dabei wurde der Quench als Apparat gerechnet, in dem thermodynamisches Gleichgewicht zwischen Gas- und Flüssigphase herrscht. Neben den Reinstoffdaten der beteiligten Komponenten wurden bei der Berechnung reale Binärdaten verwendet. Derartige Berechnungen können mit kommerziellen Rechenprogrammen, hier: Aspen Plus, die dem Fachmann geläufig sind, durchgeführt werden).

### Beispiel 2 (alternative Hydriereinstellung):

Eine Mischung bestehend aus 27 Gew.-% IPDN und 73 Gew.-% NMP, die aus den reinen Komponenten zusammengemischt wurde, wurde in einem kontinuierlich betriebenen 70 ml-Rohrreaktor an einem Kobalt-Vollkontakt bei 80°C und 190 bar hydriert. Über den Katalysator wurden stündlich 70 g IPDN-Lösung sowie 90 g Ammoniak geleitet. Die Ausbeute an MXDA betrug 96 % bezogen auf eingesetztes IPDN.

In anschließenden Destillationsschritten werden zuerst Ammoniak und danach NMP und Leichtsiedende Nebenkomponenten abgetrennt. Nach Abtrennung der hochsiedenden Verunreinigungen wird MXDA in einer Reinheit von mehr als 99,9 Gew.-% erhalten.

### Beispiel 3 (alternative Hydriereinstellung):

Eine Mischung bestehend aus 27 Gew.-% IPDN und 73 Gew.-% NMP, die aus den reinen Komponenten zusammengemischt wurde, wurde in einem kontinuierlich betriebenen 70 ml-Rohrreaktor an einem Kobalt-Vollkontakt bei 80°C und 190 bar hydriert. Über den Katalysator wurden stündlich 70 g IPDN-Lösung sowie 54 g Ammoniak geleitet. Die gleiche Volumenmenge wird als Lösemittel im Kreis gefahren. Die Ausbeute an MXDA betrug 95,5 % bezogen auf eingesetztes IPDN.

### Beispiel 4 (alternative Hydriereinstellung):

Eine Mischung bestehend aus 15 Gew.-% IPDN und 85 Gew.-% NMP, die aus den reinen Komponenten zusammengemischt wurde, wurde in einem kontinuierlich betriebenen 70 ml-Rohrreaktor an einem Kobalt-Vollkontakt bei 80°C und 190 bar hydriert. Über den Katalysator wurden stündlich 140 g IPDN-Lösung sowie 72 g Ammoniak geleitet. Die Ausbeute an MXDA betrug 96 % bezogen auf eingesetztes IPDN.

### Beispiel 5 (alternative Hydriereinstellung):

30 g IPDN sowie 5 g Raney-Nickel wurden im Rührautoklaven vorgelegt. Nach Zugabe von 66 g Ammoniak wurden 50 bar Wasserstoff aufgepresst und auf 100°C aufgeheizt. Durch Wasserstoffnachpressen wurde ein Gesamtdruck von 100 bar für 5 Stunden gehalten. Die Umsetzung von IPDN war quantitativ, wobei eine Ausbeute von 94 % bezogen auf eingesetztes IPDN erhalten wurde.

### Beispiel 6:

Die Ammonoxidation, das Quenchen sowie die Hochsiederabtrennung wurden wie im Beispiel 1 durchgeführt. Für die Lösungsmittelabtrennung wird jedoch so gefahren, dass über Sumpf bei 126°C IPDN in NMP abgezogen wird (35 Gew.-% IPDN, 62 Gew.-% NMP, ca. 3 % Tolunitril). Über Kopf werden der Rest des Lösungsmittels und der leichtsiedenden Nebenkomponenten abgetrennt.

(Die Lösungsmittelabtrennung ist das Ergebnis einer thermodynamischen Simulation wie oben beschrieben).

### Beispiel 7:

Untersuchungen zur Löslichkeit von IPDN in verschiedenen Lösungsmitteln

Die Löslichkeit von IPDN in NMP beträgt bei 60 °C ca. 26 Gew.-% und bei 90°C ca. 41 Gew.-%.
Pseudocumol erreicht bei 90 °C lediglich eine Löslichkeit von 20 Gew.-% und Mesitylen lediglich von 12 Gew.-%.
Bei 60°C liegt die Löslichkeit von IPDN in Mesitylen oder Pseudocumol jeweils unter 10 Gew.-%.

### Beispiel 8:

### Ammonoxidation von m-Xylol, anschließendes Quenchen der Reaktionsgase mit NMP als Lösungsmittel

Ein Katalysator der Zusammensetzung V₄Sb₃K_{0.4}Ba_{0.2} auf Steatit wurde als Festbett in einen Rohrreaktor eingebaut. Die Apparatur wurde von außen auf 415°C aufgeheizt. Dem Reaktor wurde verdampftes m-Xylol, gasförmiger Ammoniak und Luft zugefahren (NH₃ / m-Xylol = 14 mol / 1 mol; O₂ / m-Xylol = 4 mol / 1 mol). Der Katalysator der ersten Hälfte des Reaktors war mit 70 Gew.-% Steatitkugeln verdünnt, die zweite Hälfte mit 40 Gew.%. Im Reaktor herrschte ein leichter Überdruck von 0,02 bar. Die Hot-Spot-Temperatur erreichte 430°C. Man erhielt bei einem Umsatz von m-Xylol von 88 % eine Selektivität zu IPDN von 71 %.

Das aus dem Reaktor austretende Gasgemisch wird in einer Kolonne mit NMP gequencht. Aus der Quenchkolonne wird bei 120 °C und 1,02 bar (abs.) eine Lösung von IPDN in NMP ausgetragen, welche 0,25 Gew.-% m-Xylol, 1,3 Gew.-% Wasser, 3,6 Gew.-% Tolunitril, 27 Gew.-% IPDN und ca. 67,7 Gew.-% NMP enthält. Über Kopf der Quenchkolonne werden nicht umgesetzte Reaktionsgase und Inertgase sowie nicht umgesetztes m-Xylol sowie etwas NMP gasförmig abgezogen. Dieses Gas kann aufgearbeitet werden, um die Wertstoffe (insbesondere NH₃, m-Xylol, NMP sowie Tolunitril) in die Reaktionsstufe bzw. in den Quenchkreis zurückzuführen. Inerte und Begleitkomponenten (H₂O, N₂, CO₂, etc.) werden aus der Aufarbeitungsstufe ausgeschleust.

(Die oben angegebenen Daten des Quenchschrittes sind die Ergebnisse einer thermodynamischen Simulation, durchgeführt wie bei Beispiel 1).

### Beispiel 9:

Eine Lösung entsprechend der berechneten Zusammensetzung am Quenchaustritt, bestehend aus 0,44 g m-Xylol, 1,7 g Wasser, 0,88 g Benzonitril, 3,1 g Tolunitril, 24 g IPDN und 58 g NMP, wurde aus den reinen Komponenten zusammengemischt und der Hydrierung zugeführt. Zur Hydrierung wurde der Mischung flüssiges NH₃ zudosiert (NH₃ / IPDN =14 mol / 1 mol). Die Hydrierung erfolgte in Gegenwart von H₂ und 5 g Raney-Nickel-Katalysator bei 100 °C und einem Druck von 100 bar in einem Rührautoklaven.
Die Umsetzung von IPDN war quantitativ, wobei eine Ausbeute an MXDA von 92 % bezogen auf eingesetztes IPDN erhalten wurde.

## Patentansprüche

1. Verfahren zur Herstellung von meta-Xylylendiamin umfassend die Schritte Ammonoxidation von meta-Xylol zu Isophthalodinitril, wobei das dampfförmige Produkt dieser Ammonoxidationsstufe direkt mit einem flüssigen organischen Lösungsmittel in Kontakt gebracht wird (Quench),
Abtrennung von Produkten mit einem Siedepunkt höher als Isophthalodinitril (Hochsiedern) aus der erhaltenen Quenchlösung oder-suspension und
Hydrierung des lsophthalodinitrils,
**dadurch gekennzeichnet, dass** es sich bei dem für den Quench verwendeten organischen Lösungsmittel um N-Methyl-2-pyrrolidon (NMP) handelt,
nach der Abtrennung der Hochsieder und vor der Hydrierung eine teilweise oder vollständige Abtrennung des NMPs und/oder von Produkten mit einem Siedepunkt niedriger als Isophthalodinitril (Leichtsieder) erfolgt und
das Isophthalodinitril für den Hydrierschritt in einem organischen Lösungsmittel oder in flüssigem Ammoniak gelöst oder suspendiert ist.

2. Verfahren nach Anspruch 1, **dadurch gekennzeichnet, dass** die Abtrennung von Hochsiedern aus der erhaltenen Quenchlösung oder -suspension destillativ über Sumpf erfolgt, während Isophthalodinitril zusammen mit dem Lösungsmittel NMP und Leichtsiedern über Kopf abgetrennt werden.

3. Verfahren nach einem der vorhergehenden Ansprüche, **dadurch gekennzeichnet, dass** nach der Abtrennung der Hochsieder die teilweise oder vollständige Abtrennung des NMPs und/oder die Abtrennung von Leichtsiedern destillativ über Kopf erfolgt.

4. Verfahren nach Anspruch 1, **dadurch gekennzeichnet, dass** die Auftrennung der erhaltenen Quenchlösung oder -suspension in Hochsieder, Leichtsieder und NMP und Isophthalodinitril in einer Seitenabzugskolonne so erfolgt, dass Hochsieder über Sumpf, NMP und/oder Leichtsieder über Kopf und Isophthalodinitril über einen Seitenabzug abgetrennt werden.

5. Verfahren nach Anspruch 1, **dadurch gekennzeichnet, dass** die Auftrennung der erhaltenen Quenchlösung oder -suspension in Hochsieder, Leichtsieder und NMP und Isophthalodinitril in einer Trennwandkolonne so erfolgt, dass Hochsieder über Sumpf , NMP und/oder Leichtsieder über Kopf und Isophthalodinitril über einen Seitenabzug im Trennwandbereich der Kolonne abgetrennt werden.

6. Verfahren nach einem der vorhergehenden Ansprüche, **dadurch gekennzeichnet, dass** das Isophthalodinitril für den Hydrierschritt in NMP, Xylol, Benzylamin, Tolylamin und/oder Xylylendiamin gelöst oder suspendiert ist.

7. Verfahren nach einem der vorhergehenden Ansprüche, **dadurch gekennzeichnet, dass** die Hydrierung in Gegenwart von Ammoniak durchgeführt wird.

8. Verfahren nach einem der vorhergehenden Ansprüche, **dadurch gekennzeichnet, dass** die Ammonoxidation bei Temperaturen von 300 bis 500°C an einem Katalysator enthaltend V, Sb und/oder Cr, als Vollkatalysator oder auf einem inerten Träger, durchgeführt wird.

9. Verfahren nach einem der vorhergehenden Ansprüche, **dadurch gekennzeichnet, dass** bei dem Quench mit NMP die Temperatur des Quenchaustrags 40 bis 180°C beträgt.

10. Verfahren nach einem der vorhergehenden Ansprüche, **dadurch gekennzeichnet, dass** die Hydrierung bei Temperaturen von 40 bis 150°C an einem Katalysator enthaltend Ni, Co und/oder Fe, als Vollkatalysator oder auf einem inerten Träger, durchgeführt wird.

11. Verfahren nach einem der vorhergehenden Ansprüche, **dadurch gekennzeichnet, dass** nach der Hydrierung eine Reinigung des meta-Xylylendiamins durch Abdestillation des gegebenenfalls eingesetzten Lösungsmittels und Ammoniaks sowie gegebenenfalls leichtersiedender Nebenprodukte über Kopf und destillativer Abtrennung von schwerersiedenden Verunreinigungen über Sumpf erfolgt.

12. Verfahren nach einem der vorhergehenden Ansprüche, **dadurch gekennzeichnet, dass** man nach der Hydrierung das gegebenenfalls eingesetzte Lösungsmittel und Ammoniak sowie gegebenenfalls leichtsiedende Nebenprodukte abdestilliert und danach meta-Xylylendiamin destillativ von schwersiedenden Verunreinigungen abtrennt.

13. Verfahren nach einem der beiden vorhergehenden Ansprüche, **dadurch gekennzeichnet, dass** das meta-Xylylendiamin nach der Destillation zur weiteren Reinigung mit einem organischem Lösungsmittel extrahiert wird.

14. Verfahren nach dem vorhergehenden Anspruch, **dadurch gekennzeichnet, dass** man zur Extraktion Cyclohexan oder Methylcyclohexan verwendet.

## Claims

1. A process for preparing meta-xylylenediamine, comprising the steps of ammoxidizing meta-xylene to isophthalonitrile, by contacting the vaporous product of this ammoxidation stage directly with a liquid organic solvent (quench),
removing products having a boiling point higher than isophthalonitrile (high boilers) from the resulting quench solution or suspension and hydrogenating the isophthalonitrile,
wherein the organic solvent used for the quench is N-methyl-2-pyrrolidone (NMP),
after the removal of the high boilers and before the hydrogenation, there is a partial or complete removal of the NMP and/or of products having a boiling point lower than isophthalonitrile (low boilers) and
the isophthalonitrile for the hydrogenation step is dissolved or suspended in an organic solvent or in liquid ammonia.

2. The process according to claim 1, wherein the high boilers are removed distillatively from the resulting quench solution or suspension via the bottom, while isophthalonitrile is removed via the top together with the NMP solvent and low boilers.

3. The process according to any of the preceding claims, wherein, after the removal of the high boilers, the NMP is removed partly or completely and/or low boilers are removed distillatively via the top.

4. The process according to claim 1 wherein the resulting quench solution or suspension is separated into high boilers, low boilers and NMP isophthalonitrile in a sidestream column in such a way that high boilers are removed via the bottom, NMP and/or low boilers via the top and isophthalonitrile via a sidestream.

5. The process according to claim 1, wherein the resulting quench solution or suspension is separated into high boilers, low boilers and NMP and isophthalonitrile in a dividing wall column in such a way that high boilers are removed via the bottom, NMP and/or low boilers via the top and isophthalonitrile via a sidestream in the dividing wall region of the column.

6. The process according to any of the preceding claims, wherein the isophthalonitrile for the hydrogenation step is dissolved or suspended in NMP, xylene, benzylamine, tolylamine and/or xylylenediamine.

7. The process according to any of the preceding claims, wherein the hydrogenation is carried out in the presence of ammonia.

8. The process according to any of the preceding claims, wherein the ammoxidation is carried out at temperatures of from 300 to 500°C over a catalyst comprising V, Sb and/or Cr, as an unsupported catalyst or on an inert support.

9. The process according to any of the preceding claims, wherein the temperature of the quench effluent in the quench with NMP is from 40 to 180°C.

10. The process according to any of the preceding claims, wherein the hydrogenation is carried out at temperatures of from 40 to 150°C over a catalyst comprising Ni, Co and/or Fe, as an unsupported catalyst or on an inert support.

11. The process according to any of the preceding claims, wherein, after the hydrogenation, the meta-xylylenediamine is purified by distilling off any solvent used and ammonia, and also any relatively low-boiling by-products, via the top and distillatively removing relatively high-boiling impurities via the bottom.

12. The process according to any of the preceding claims, wherein, after the hydrogenation, any solvent used and ammonia and also any low-boiling by-products, are distilled off and, afterwards, meta-xylylenediamine is removed from high-boiling impurities by distillation.

13. The process according to either of the two preceding claims, wherein the meta-xylylenediamine, after the distillation, is extracted for further purification with an organic solvent.

14. The process according to the preceding claim, wherein cyclohexane or methylcyclohexane are used for the extraction.

## Revendications

1. Procédé de préparation de méta-xylylènediamine comportant les étapes
d'ammonoxydation de méta-xylène en isophtalodinitrile, le produit en forme de vapeur de cette étape d'ammonoxydation étant directement amené en contact avec un solvant organique liquide (refroidissement brusque),
d'isolement de produits ayant un point d'ébullition supérieur à l'isophtalodinitrile (substances à haut point d'ébullition) à partir de la solution ou suspension de refroidissement brusque obtenue, et
d'hydrogénation de l'isophtalodinitrile,
**caractérisé en ce que**, en ce qui concerne le solvant organique utilisé pour le refroidissement brusque, il s'agit de N-méthyl-2-pyrrolidone (NMP),
un isolement partiel ou complet de la NMP et/ou des produits ayant un point d'ébullition plus bas que l'isophtalodinitrile (substances à bas point d'ébullition) a lieu après l'isolement des substances à haut point d'ébullition et avant l'hydrogénation, et étant l'isophtalodinitrile est, pour l'étape d'hydrogénation, dissous ou amené en suspension dans un solvant organique ou dans de l'ammoniac liquide.

2. Procédé suivant la revendication 1, **caractérisé en ce que** l'isolement des substances à haut point d'ébullition à partir de la solution ou suspension de refroidissement brusque obtenue a lieu par distillation, par l'intermédiaire du fond de colonne, tandis que l'isophtalodinitrile est sépare conjointement au solvant NMP et aux substances à bas point d'ébullition par l'intermédiaire de la tête.

3. Procédé suivant l'une des revendications précédentes, **caractérisé en ce que**, après l'isolement des substances à haut point d'ébullition, la séparation partielle ou complète de la NMP et/ou la séparation des substances à bas point d'ébullition a lieu par distillation par l'intermédiaire de la tête.

4. Procédé suivant la revendication 1, **caractérisé en ce que** la séparation de la solution ou suspension de refroidissement brusque obtenue en substances à haut point d'ébullition, substances à bas point d'ébullition et NMP et isophtalodinitrile a lieu dans une colonne à soutirage latéral de façon que les substances à haut point d'ébullition soient séparées par le fond de colonne, la NMP et/ou les substances à bas point d'ébullition par la tête et l'isophtalodinitrile par soutirage latéral.

5. Procédé suivant la revendication 1, **caractérisé en ce que** la séparation de la solution ou suspension de refroidissement brusque obtenue en substances à haut point d'ébullition, substances à bas point d'ébullition et NMP et isophtalodinitrile a lieu dans une colonne à paroi séparatrice de façon que les substances à haut point d'ébullition soient séparées par le fond de colonne, la NMP et/ou les substances à bas point d'ébullition par la tête et l'isophtalodinitrile par un soutirage latéral dans la zone de paroi séparatrice de la colonne.

6. Procédé suivant l'une des revendications précédentes, **caractérisé en ce que** l'isophtalodinitrile est, pour l'étape d'hydrogénation, dissous ou amené en suspension dans de la NMP, du xylène, de la benzylamine, de la tolylamine et/ou de la xylylènediamine.

7. Procédé suivant l'une des revendications précédentes, **caractérisé en ce que** l'hydrogénation est effectuée en présence d'ammoniac.

8. Procédé suivant l'une des revendications précédentes, **caractérisé en ce que** l'ammonoxydation est effectuée à des températures de 300 à 500°C sur un catalyseur contenant V, Sb et/ou Cr, sous la forme de catalyseur massique ou sur un support inerte.

9. Procédé suivant l'une des revendications précédentes, **caractérisé en ce que**, lors du refroidissement brusque avec la NMP, la température de l'effluent est de 40 à 180°C.

10. Procédé suivant l'une des revendications précédentes, **caractérisé en ce que** l'hydrogénation est effectuée à des températures de 40 à 150°C sur un catalyseur contenant Ni, Co et/ou Fe, sous la forme d'un catalyseur massique ou sur un support inerte.

11. Procédé suivant l'une des revendications précédentes, **caractérisé en ce que**, après l'hydrogénation, une purification de la méta-xylylènediamine a lieu par distillation du solvant et de l'ammoniac éventuellement mis en oeuvre ainsi qu'éventuellement des produits secondaires à bas point d'ébullition, par l'intermédiaire de la tête, et une séparation par distillation d'impuretés à haut point d'ébullition a lieu par le fond de colonne.

12. Procédé suivant l'une des revendications précédentes, **caractérisé en ce que**, après l'hydrogénation, on sépare par distillation le solvant et l'ammoniac éventuellement mis en oeuvre ainsi qu'éventuellement des produits secondaires à faible point d'ébullition et ensuite on sépare par distillation de la méta-xylylènediamine des impuretés à point d'ébullition élevé.

13. Procédé suivant l'une des deux revendications précédentes, **caractérisé en ce que** la méta-xylylènediamine est, après la distillation, extraite par un solvant organique pour réaliser une purification plus poussée.

14. Procédé suivant la revendication précédente, **caractérisé en ce que**, pour l'extraction, on utilise du cyclohexane ou du méthylcyclohexane.
